# EUROPEAN PATENT APPLICATION

(11) **EP 4 843 517 A1**
(43) Date of publication of application: **25.11.2026**
(21) Application number: 25186488.0
(22) Date of filing: 30.06.2025
(51) Int. Cl.: A61B 17/15, A61B 17/17

(54) **SURGICAL GUIDE DEVICE FOR ORTHOGNATHIC SURGERY**

(30) Priority: 19.05.2025 US 202519211653
(71) Applicant: Techfit Digital Surgery Inc., Daytona Beach, Florida 32114 (US)
(72) Inventor: TORO RESTREPO, Mauricio, Daytona Beach, Florida, 32114 (US); OCAMPO LÓPEZ, Valentina, Envigado, Antioquia (CO)
(74) Representative: Boult Wade Tennant LLP

(57) **Abstract**

A surgical guide for orthognathic surgery made up of two parts is provided. A first part includes a splint made of resin through the 3D printing method, where the splint is designed to fit the geometry of the patient's dentition and includes a bridge that connects to the second part of the guide, which is a plate made of titanium through subtractive manufacturing. In this configuration, the bridge of the splint has a "C" shaped geometry that fits with the anvil geometry included in the plate. Furthermore, the bridge features a reduced area portion that allows it to break once the procedure is completed.

## Description

### Field of technology

The following relates to the field of maxillofacial surgery, and more particularly to a surgical guide for orthognathic surgery composed of two parts. A first part comprises a splint made of resin using 3D printing technology, wherein the splint is designed to fit the geometry of the patient's dentition and includes a bridge that engages with the second part of the guide, which consists of or comprises a plate manufactured from titanium via subtractive manufacturing. The bridge of the splint features a "C"-shaped geometry that fits with an anvil-shaped geometry on the plate. The bridge includes a reduced cross-sectional area that allows for breakage once the procedure has been completed.

### Background

The growing demand for techniques that preserve soft tissues, achieve precise margins, and facilitate aesthetic rehabilitation and functional restoration presents significant challenges in orthognathic surgery of the maxillofacial region. To address these challenges, surgical guides are essential tools that enable the transfer of a predefined surgical plan to the operating room.

It is essential to achieve high accuracy in the placement of these guides onto the patient's anatomy. Furthermore, they must possess adequate rigidity and strength to effectively guide drilling and surgical cutting instruments. A key challenge lies in providing guides that offer both the necessary mechanical strength and sufficient flexibility to be precisely positioned on the patient's anatomy, ensuring proper contact with body surfaces that serve as reliable reference points for alignment.

However, traditional surgical guides present limitations that may result in excessive soft tissue displacement during surgery. Proper placement of these guides can be challenging when it is not feasible to fully remove the surrounding tissues, which can lead to misalignments and inaccuracies in the surgical procedure.

Computer-aided design and manufacturing (CAD/CAM) techniques, particularly Digital Surgical Planning (DSP), have succeeded in improving surgical accuracy and reducing operation times. However, many of the current surgical guides are bulky and rely heavily on bone support.

An alternative to traditional surgical guides is tooth-supported guides, which can help address some of the aforementioned issues. However, the manufacturing methods for these guides, whether through 3D printing or machining, do not allow for full occlusal support, generally being limited to a maximum of six teeth. For instance, during genioplasty procedures, surgeons must disassemble the previously installed anchorage to place the surgical guide, perform the genioplasty, and then reassemble. This process can be tedious and increases the likelihood of occlusal errors, negatively impacting the accuracy of the procedure. The same principle applies to other orthognathic surgeries, such as the bilateral sagittal split osteotomy (BSSO) of the mandibular ramus and the Le Fort osteotomy. In these procedures, the stability and precise fit of the guide are essential to ensure proper bone alignment and to minimize potential deviations in occlusion. Therefore, optimizing the design and manufacturing of these guides is key to improving the predictability and safety of the surgery.

Accordingly, the conventional art includes inventions such as the one disclosed in document US8764441B2, which describes a method and apparatus for skeletal positioning. It includes a splint with a main body coupled to a patient's skeletal structure and a positioning guide comprising a skeletal plate, a splint plate, and an arm connecting both plates. The skeletal plate includes at least one hole, while the splint plate has connectors to removably attach the positioning guide to the splint. The relative position and orientation of the skeletal plate and the splint plate are predetermined in order to align a portion of the skeletal plate with a defined part of the patient's skeletal structure.

However, this document presents the drawback of not specifically disclosing a bridge portion with a "C"-shaped geometry, nor a breakaway mechanism following the procedure. Additionally, it does not specify the manufacturing processes of the materials used.

The document US20220117665A1 refers to a surgical guide that includes a metal guide and a plastic reference created through additive manufacturing. The metal guide is configured to fit the bony surface of a first area of the body and has a customized main plate for the bony surface. The main plate defines mounting guides to secure it to the bony surface and provides a guide for drilling or cutting through it. An arm extends from one end of the main plate to a distal end. The plastic reference has a customized reference surface to fit the surface of a second area of the body, physically separated from the first area.

However, this document presents the drawback of not specifically disclosing a resin or titanium splint with a "C"-shaped bridge that fits into an anvil-shaped geometry. Additionally, it does not refer to a reduced cross-sectional area to facilitate breakage after the procedure.

Document WO2015065057A1 discloses a maxillary transfer guide template for orthognathic surgery and a method for its manufacture. This method is for producing a cutting guide template that accurately guides a cutting line on the upper maxilla and includes the following steps: a) generating a 3D cranial image file and a 3D dental region image file of a patient; b) integrating both images to obtain a merged image; c) trimming the temporomandibular joint from the merged image to simulate the correction; d) after the simulation, generating the shape of the cutting guide template, which consists of a main body of the template, a cutting guide slot, and a perforated part; e) manufacturing the cutting guide template using the shape data generated.

However, this document presents the drawback of not specifically disclosing materials such as resin or titanium for the guide, nor a two-part design with a "C"-shaped bridge. Additionally, WO2015065057A1 does not specify a programmed breakage design.

The document KR102180559B1 teaches a patient-specific chin surgery guide, a manufacturing method, a recording medium, and an apparatus for such manufacturing. In embodiments, the method for manufacturing the chin surgery guide involves cutting or repositioning the chin into an appropriate position and securing it properly, through the following steps: a) creating a 3D image file of the patient's skull, a 3D file of the teeth, and merging both images; b) performing a virtual surgical simulation of the chin cut and repositioning it; c) generating the shape of the chin surgery guide, including a fixation portion and a support template; d) manufacturing the chin surgery guide using the generated shape data.

However, this document presents the disadvantage of not specifically disclosing a resin splint with a titanium plate in a "C" and anvil configuration, nor does it mention the concept of a breakaway section after the surgery.

### Summary

An aspect relates to a guide device for orthognathic surgery composed of two parts: a first part being a resin splint and a second part being a guide made of titanium, which allows for the creation of a unique assembly that provides specialists with complete occlusal registration. This guide device utilizes the final, intermediate, or preoperative splint and a bridge that assembles to the titanium guide portion in contact with the bone. This titanium guide portion should be as small as possible to minimize the need to resect excess soft tissue. It offers maximum stability and precision, facilitating the procedure with a less invasive incision and technique. Additionally, it allows the surgeon to maintain the final splint after completing the mentoplasty, avoiding readjustments and saving time in the process.

### Brief description

Some of the embodiments will be described in detail, with references to the following Figures, wherein like designations denote like members, wherein:

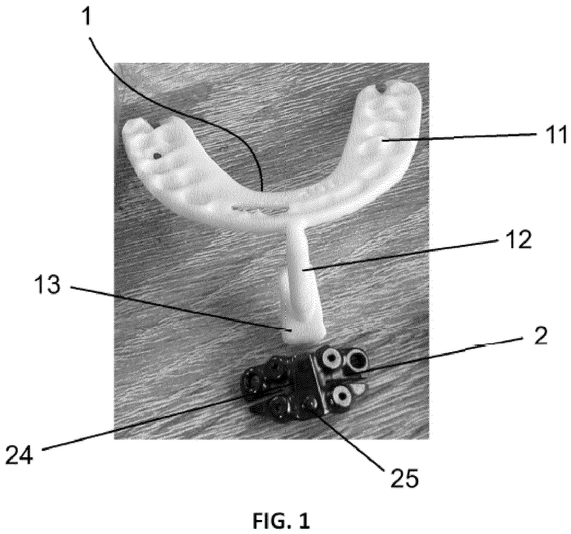

Figure 1 shows a view of the surgical guide device for orthognathic surgery of embodiments of the present invention, highlighting the two parts that constitute it: the splint and the guide;

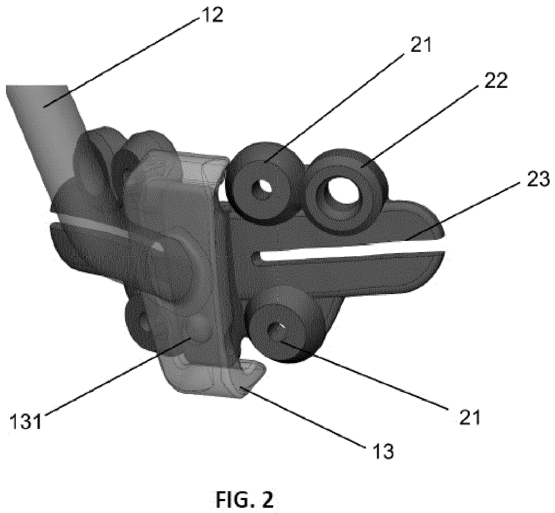

Figure 2 provides a detailed view of the bridge and the guide according to embodiments of the present invention;

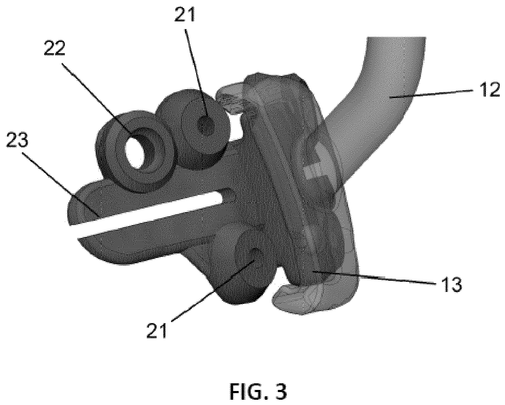

Figure 3 shows a detailed view of the bridge and the guide according to embodiments of the present invention;

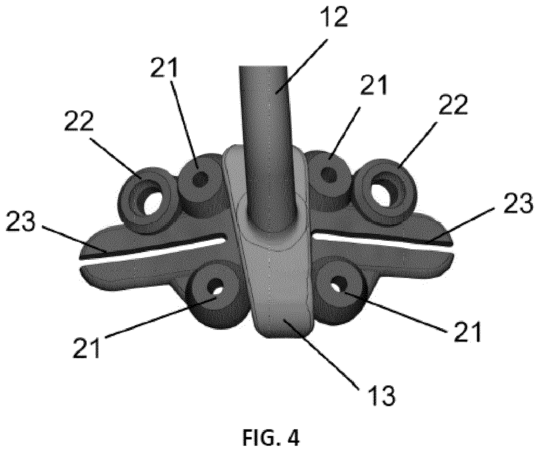

Figure 4 shows a detailed view of the bridge and the guide according to embodiments of the present invention;

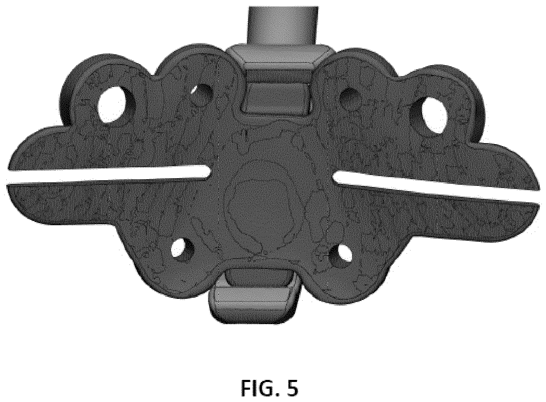

Figure 5 provides a detailed view of the posterior surface of the guide according to embodiments of the present invention; and

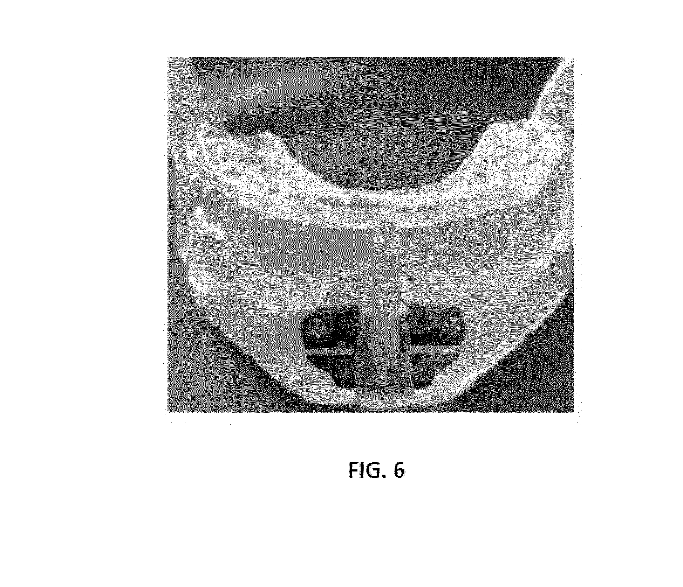

Figure 6 shows a view of the surgical guide device for orthognathic surgery of embodiments of the present invention in one of its possible applications.

### Detailed description

Embodiments of the present invention relate to a surgical guide device for orthognathic surgery, formed by two parts: a first part consisting of a splint (1) and a second part consisting of a guide (2). The splint (1) is formed by an arch-shaped geometry (11), a bridge (12), and a "C"-shaped fastening mechanism (13). The guide (2) is a metal plate that includes fixation holes (21), drilling holes (22), cutting slots (23), an anvil (24), and a male hemispherical tooth (25).

Wherein, the splint may be a final, intermediate, or preoperative splint that records all of the patient's upper and lower teeth and is custom-made for the patient. In an embodiment, the splint records the patient's upper and lower teeth from the incisors to the midpoint of the second molar. The splint is made of resin or biocompatible polymers, using additive manufacturing or 3D printing methods.

In an embodiment, the splint has a thickness between 2.5 mm and 3 mm, a vestibular contour of 1 mm, and includes wiring holes.

Wherein, the bridge (12) connects the arcuate geometry (11) of the splint (1) with the "C" shaped fastening mechanism (13). The bridge (12) is a cylindrical arm that includes a reduction in cross-sectional area at the point where it connects with the arcuate geometry (11). The bridge (12) has a uniform thickness of 3 mm, except at the point where it connects with the arcuate geometry (11), where the thickness is 2 mm. This design allows the specialist to break the bridge and release the final splint upon completion of the procedure.

Wherein the "C" shaped fastening mechanism (13) includes a female hemispherical tooth (131) that aligns with the male hemispherical tooth (25) of the guide (2) when assembled with the anvil (24).

Wherein the guide (2) is made of titanium or surgical steel or by a subtractive manufacturing method, with the lower surface of the guide (2) being custom-fabricated to match the patient's bone composition. The guide (2) has at least four perforation holes (22), two in the upper part and two in the lower part, as well as at least two fixation holes (21) on the upper distal part, two lateral cutting grooves (23) that indicate the cut, and in the center of the guide is the anvil (24) which includes a male hemispherical tooth (25) that interacts with the "C" shaped fastening mechanism (13) of the splint (1). Wherein the guide (2) is symmetrical with respect to the axis passing through the anvil (24).

The assembly between the splint (1) and the guide (2) is performed by aligning the anvil (24) and the "C" shaped fastening mechanism (13) until a clicking sound is heard, indicating that the two parts are securely engaged.

The described surgical guide device for orthognathic surgery offers the following key benefits:
- Occlusal Fixation: By being fixed to the splint, the device allows for the best possible occlusal record, providing optimal stability and precision during surgery.
- Reduction in Surgical Time: The integration of the cutting guide with the splint eliminates the need for using two separate devices, optimizing the procedure and eliminating the need for double cerclage, thus reducing the total time of the intervention.
- Customized Design: Precise manufacturing, along with advanced surgical planning, ensures an exact anatomical fit, enhancing the adaptability of the guide and reducing the risk of complications during and after surgery.
- Reduced Trauma: With the incorporated occlusal support, a bone guide can be designed to be 60% smaller, enabling a smaller incision, reducing soft tissue resections, and contributing to a quicker, less traumatic recovery for the patient.

On the other hand, embodiments of the present invention can be better understood from the following definitions:

Splint: A rigid or semi-rigid device, typically made from acrylic, resin, or polymer materials, designed to ensure the stability and correct position of anatomical structures after a surgical or rehabilitative procedure. In the context of maxillofacial or orthognathic surgery, the splint is used to transfer pre-surgical planning to the desired position of the maxilla and mandible, ensuring the precision of the outcome.

Preoperative Splint: The preoperative splint is a device used in surgical planning, attached to the patient's preoperative position. In embodiments of the present invention, this splint may be connected to the chin surgical guide device of embodiments of the present invention, BSSO, or Le Fort.

Intermediate Splint: The intermediate splint is used during surgery to help correctly position the mandible or maxilla in procedures requiring sequential movement of bone segments, such as in bimaxillary surgery. Its main function is to guide the new position of one of the bone segments before the final adjustment is made.

Final Splint: The final splint is used in the final phase of surgery to ensure that the bone segments are in the planned definitive position, guaranteeing correct occlusion and skeletal alignment. Its design is crucial to avoid errors in bone fixation and ensure adequate stability in the postoperative period. The chin surgical guide device of embodiments of the present invention is the most commonly used.

Surgical Guide: A personalized instrument that assists surgeons in making precise cuts or perforations during a surgical procedure. These guides are designed from imaging studies and 3D planning. In orthognathic or maxillofacial surgery, surgical guides are used to properly position surgical instruments (such as saws or drills) on bone structures, minimizing errors and optimizing outcomes.

3D Planning: The digital process of designing and simulating surgery in a three-dimensional environment, using specialized software that allows manipulation of images obtained from computed tomography (DICOM) scans or intraoral scans. 3D planning facilitates detailed anatomical evaluation, the creation of virtual models, and the simulation of surgical movements or interventions before performing them on the patient. In maxillofacial surgery, this method improves accuracy, reduces surgical time, and optimizes functional and aesthetic results.

Although the present invention has been disclosed in the form of embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements.

## Claims

1. A surgical guide device for orthognathic surgery comprises two parts, a first part comprising a splint and a second part comprising a guide, where the splint is formed by an arc-shaped geometry, a bridge, and a "C" shaped fastening mechanism, and where the guide is a metallic plate that includes fixation holes, perforation holes, cutting slots, an anvil, and a male semi-spherical geometry.

2. The surgical guide device for orthognathic surgery according to claim 1, wherein the splint can be a final, intermediate, or preoperative splint that registers the patient's upper and lower teeth and is made of biocompatible resin or polymer tailored to the patient, by an additive manufacturing method.

3. The surgical guide device for orthognathic surgery according to claim 1, wherein the splint registers the patient's upper and lower teeth from the incisors to the midpoint of the second molar.

4. The surgical guide device for orthognathic surgery according to claim 1, wherein the splint has a thickness between 2.5 mm and 3 mm, a vestibular contour of 1 mm, and includes wiring holes.

5. The surgical guide device for orthognathic surgery according to claim 1, wherein the bridge connects the arc-shaped geometry of the splint with the "C" shaped fastening mechanism, where the bridge is a cylindrical arm that includes a reduction in cross-sectional area at the point where it connects with the arc-shaped geometry.

6. The surgical guide device for orthognathic surgery according to claim 1, wherein the bridge has a uniform thickness of 3 mm, except at the point where it connects with the arc-shaped geometry, where the thickness is 2 mm.

7. The surgical guide device for orthognathic surgery according to claim 1, wherein the "C" shaped fastening mechanism has a female semi-spherical tooth that aligns with the male semi-spherical tooth of the guide when assembled with the anvil.

8. The surgical guide device for orthognathic surgery according to claim 1, wherein the guide is made of titanium or surgical steel by a subtractive manufacturing method, with the lower surface of the guide custom-made to the patient according to their bone composition.

9. The surgical guide device for orthognathic surgery according to claim 1, wherein the guide has at least four perforation holes, two in the upper part and two in the lower part, in addition to having at least two fixation holes in the distal upper part, two lateral cutting slots that indicate the cut, and at the center of the guide is the anvil which includes a male semi-spherical tooth that interacts with the "C" shaped fastening mechanism of the splint.

10. The surgical guide device for orthognathic surgery according to claim 1, wherein the guide is symmetrical with respect to the axis passing through the anvil.
